# EUROPEAN PATENT SPECIFICATION

(11) **EP 4 392 555 B1**
(45) Date of publication and mention of the grant of the patent: **05.08.2026**
(21) Application number: 22862222.1
(22) Date of filing: 23.08.2022
(51) Int. Cl.: C12N 15/10, C12P 19/34, C12Q 1/6806, C12Q 1/6827, C12Q 1/6855

(54) **METHODS AND COMPOSITIONS FOR DETECTING GENOMIC METHYLATION**
VERFAHREN UND ZUSAMMENSETZUNGEN ZUM NACHWEIS GENOMISCHER METHYLIERUNG
PROCÉDÉS ET COMPOSITIONS DE DÉTECTION DE MÉTHYLATION GÉNOMIQUE

(30) Priority: 26.08.2021 US 202163237297 P
(43) Date of publication of application: 03.07.2024
(73) Proprietor: Illumina, Inc., San Diego, CA 92122 (US)
(72) Inventor: DESANTIS, Grace, San Diego, California 92122 (US); KENNEDY, Andrew, San Diego, California 92122 (US); STEEMERS, Frank J., San Diego, California 92122 (US)
(74) Representative: Williams, Andrea
(86) International application number: PCT/US2022/075327
(87) International publication number: WO 2023/028478

(56) References cited:
- WO-A1-2009/132315
- WO-A2-2010/048337
- US-A1- 2009 075 343
- US-A1- 2015 011 396
- US-A1- 2016 177 359
- US-A1- 2016 265 042
- US-A1- 2019 177 776

## Description

### CROSS-REFERENCE TO RELATED APPLICATIONS

This application claims priority to U.S. Prov. App. No. 63/237297, Filed August 26, 2021.

### REFERENCE TO SEQUENCE LISTING

The present application is being filed along with a Sequence Listing in electronic format. The Sequence Listing is provided as a file entitled ILLINC566WOSEQ, created August 16, 2022, which is approximately 28 kilobytes in size. The information in the electronic format of the Sequence Listing is incorporated herein by reference in its entirety.

### FIELD OF THE INVENTION

Some embodiments provided herein relate to the preparation of nucleic acid libraries for detecting genomic methylation. Some embodiments include the use of hairpin adapters to physically link a conversion-sensitive strand with a conversion-resistant strand. Some embodiments include the use of adapters comprising tags such that a sequence derived from a template strand can be matched with a sequence derived from the complementary strand of the nucleic acid of the sample.

### BACKGROUND OF THE INVENTION

Biomolecule methylation, such as DNA methylation is widespread and plays a critical role in the regulation of gene expression in development, differentiation and disease. Methylation in particular regions of genes, for example their promoter regions, can inhibit the expression of these genes. Earlier work has shown that the gene silencing effect of methylated regions is accomplished through the interaction of methylcytosine binding proteins with other structural components of the chromatin, which, in turn, makes the DNA inaccessible to transcription factors through histone deacetylation and chromatin structure changes. Genomic imprinting in which imprinted genes are preferentially expressed from either the maternal or paternal allele also involves DNA methylation. Deregulation of imprinting has been implicated in several developmental disorders.

In vertebrates, the DNA methylation pattern is established early in embryonic development and in general the distribution of 5-methylcytosine (5 mC) along the chromosome is maintained during the life span of the organism. Stable transcriptional silencing is critical for normal development and is associated with several epigenetic modifications. If methylation patterns are not properly established or maintained, various disorders like mental retardation, immune deficiency and sporadic or inherited cancers may follow. The study of methylation is particularly pertinent to cancer research as molecular alterations during malignancy may result from a local hypermethylation of tumor suppressor genes, along with a genome wide demethylation.

The initiation and the maintenance of the inactive X-chromosome in female eutherians were found to depend on methylation. Rett syndrome (RTT) is an X-linked dominant disease caused by mutation of MeCP2 gene, which is further complicated by X-chromosome inactivation (XCI) pattern. The current model predicts that MeCP2 represses transcription by binding methylated CpG residues and mediating chromatin remodeling.

DNA methylation pattern changes at certain genes often alter their expression, which could lead to cancer metastasis, for example. Thus, studies of methylation pattern in selected, staged tumor samples compared to matched normal tissues from the same patient offers a novel approach to identify unique molecular markers for cancer classification. Monitoring global changes in methylation pattern has been applied to molecular classification in breast cancer. In addition, many studies have identified a few specific methylation patterns in tumor suppressor genes (for example, p16, a cyclin-dependent kinase inhibitor) in certain human cancer types.

Restriction landmark genomic scanning (RLGS) profiling of methylation pattern of 1184 CpG islands in 98 primary human tumors revealed that the total number of methylated sites is variable between and in some cases within different tumor types, suggesting there may be methylation subtypes within tumors having similar histology. Aberrant methylation of a proportion of these genes correlates with loss of gene expression.

Since genomic DNA is often the target of methylation analyses, it offers advantages in both the availability of the source materials and ease of performing such analyses. Also, methylation analyses of genomic DNA can be complementary to those used for RNA-based gene expression profiling.

WO 2010/048337 A2 discloses ligation of hairpin adapters to a double stranded template followed by denaturation to separate the top and bottom strands from each other. This is followed by extension of the 3' end of the hairpin in the presence of methylated dCTP resulting in linked strands between the template and the complementary strand comprising conversion resistant cytosines.

WO 2009/132315 A1 discloses methods of determining the methylation profile of a target nucleic acid comprising ligating the 5' end of a hairpin adapter to the target nucleic acid so that there is a nick at the 3' end of the adapter which is extended in the presence of 5- methyl-dCTP to form a fully methylated strand. This is followed by ligation to Y-shaped biotinylated adapters, capture and sequencing. After denaturation, the ligated strand of the adapter is hybridized to a primer which is extended in the presence of conversion resistant cytosines. Accordingly, there is a need for improved methods of determining the methylation status of DNA. The compositions, methods and systems described herein satisfy this need and provide other advantages as well.

### SUMMARY OF THE INVENTION

Some embodiments of the methods and compositions provided herein include a method of preparing a nucleic acid library, comprising: (a) obtaining a plurality of double-stranded template nucleic acids, and a first adapter comprising a hairpin and a double-stranded region comprising a nick or a nickable site, wherein the nickable site comprises a uracil or a ribonucleotide; (b) ligating the first adapter to each end of the double-stranded template nucleic acids by double-stranded ligation; (c) denaturing the double-stranded template nucleic acids to obtain single-stranded template nucleic acids comprising a hairpin; (d) extending the hairpin in the presence of a conversion-resistant cytosine analog to obtain extended hairpins comprising a template strand and a complementary strand; and (e) ligating a Y-adapter to an end of the extended hairpins by double-stranded ligation to obtain library polynucleotides.

Not encompassed by the wording of the claims, some embodiments also include converting conversion-sensitive cytosine residues of the library polynucleotides to another base residue to obtain converted polynucleotides. In some embodiments, the converting comprises bisulfite conversion.

In some embodiments, the double-stranded region of the first adapter comprises the nickable site.

In some embodiments, (c) comprises contacting the nickable site with an enzyme prior to the denaturing, wherein the enzyme is selected from a uracil DNA glycosylase (UDG), a DNA glycosylase-lyase, an RNase H, or a combination thereof.

In some embodiments, the Y-adapter comprises a double-stranded portion and a non-complementary portion comprising a first single strand and a second single strand.

In some embodiments, the first single strand and/or the second single strand comprise a sequencing primer binding site, an amplification primer binding site, and/or a target site for a capture probe.

Not encompassed by the wording of the claims, some embodiments also include (i) amplifying the library polynucleotides or converted polynucleotides; and/or (ii) adding indexes to the library polynucleotides or converted polynucleotides.

In some embodiments, the double-stranded region of the first adapter comprises a tag sequence.

In some embodiments, (a) comprises obtaining a plurality of the first adapter, wherein the tag sequences of the plurality of the first adapter are different from one another.

Some embodiments also include identifying a first sequence of a converted polynucleotide and a second sequence of a converted polynucleotide comprising the same tag sequence by comparing tag sequences of the converted polynucleotides, thereby identifying a first sequence of a converted polynucleotide and a second sequence of a library polynucleotide derived from the same double-stranded template nucleic acid.

Not encompassed by the wording of the claims, in some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, some embodiments also include sequencing the converted polynucleotides. Some embodiments also include aligning sequences of the converted polynucleotides with a reference sequence. Some embodiments also include aligning a sequence of a template strand with a sequence of a complementary strand. Some embodiments also include mapping a methylated cytosine residue on a sequence of a converted polynucleotide or a reference sequence.

Not encompassed by the wording of the claims, in some embodiments, the plurality of double-stranded template nucleic acids comprises genomic DNA or cell-free DNA.

Not encompassed by the wording of the claims, some embodiments of the methods and compositions provided herein include a method of preparing a nucleic acid library, comprising: (a) obtaining a plurality of double-stranded template nucleic acids, and a first adapter comprising a first adapter strand and second adapter strand, wherein the first adapter strand comprises a hairpin and a double-stranded region formed between a 5' end of the first adapter strand and a 3' end of the second adapter strand, wherein a 5' end of the second adapter strand is single-stranded; (b) ligating the first adapter to each end of the double-stranded template nucleic acids by double-stranded ligation; (c) denaturing the double-stranded template nucleic acids to obtain single-stranded template nucleic acids; and (d) extending the hairpin in the presence of a conversion-resistant cytosine analog to obtain to obtain library polynucleotides comprising a template strand and a complementary strand.

Not encompassed by the wording of the claims, some embodiments also include converting conversion-sensitive cytosine residues of the library polynucleotides to another base residue to obtain converted polynucleotides. **In** some embodiments, the converting comprises bisulfite conversion.

Not encompassed by the wording of the claims, in some embodiments, the single-stranded 5' end of the second adapter strand comprises a sequencing primer binding site, an amplification primer binding site, and/or a target site for a capture probe.

Not encompassed by the wording of the claims, in some embodiments, the tailed primers comprise a sequencing primer binding site, an amplification primer binding site, and/or a target site for a capture probe.

Not encompassed by the wording of the claims, in some embodiments, the double-stranded region of the first adapter a sequencing primer binding site, an amplification primer binding site, and/or a target site for a capture probe.

Some embodiments also include (i) amplifying the library polynucleotides or converted polynucleotides; and/or (ii) adding indexes to the library polynucleotides or converted polynucleotides.

In some embodiments, the double-stranded region of the first adapter comprises a tag sequence, wherein
(a) comprises obtaining a plurality of the first adapter, wherein the tag sequences of the plurality of the first adapter are different from one another.

Some embodiments also include identifying a first sequence of a converted polynucleotide and a second sequence of a converted polynucleotide comprising the same tag sequence by comparing tag sequences of the converted polynucleotides, thereby identifying a first sequence of a converted polynucleotide and a second sequence of a converted polynucleotide derived from the same double-stranded template nucleic acid.

Not encompassed by the wording of the claims, in some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, some embodiments also include sequencing the converted polynucleotides. Some embodiments also include aligning sequences of the converted polynucleotides with a reference sequence. Some embodiments also include aligning a sequence of a template strand with a sequence of a complementary strand. Some embodiments also include mapping a methylated cytosine residue on a sequence of a converted polynucleotide or a reference sequence.

Not encompassed by the wording of the claims, insome embodiments, the plurality of double-stranded template nucleic acids comprises genomic DNA or cell-free DNA.

Not encompassed by the wording of the claims, some embodiments of the methods and compositions provided herein include a method of preparing a nucleic acid library, comprising: (a) obtaining a plurality of double-stranded template nucleic acids by (i) contacting double stranded DNA with a plurality of transposomes comprising a first adapter to obtain DNA fragments, and (ii) end-filling each DNA fragment, wherein each end of the double-stranded template nucleic acids comprises the first adapter; (b) denaturing the double-stranded template nucleic acids to obtain single-stranded template nucleic acids; (c) hybridizing a first tailed primer to a region at an end of the single-stranded template nucleic acids, and extending the hybridized primer in the presence of a conversion-resistant cytosine analog to obtain extended polynucleotides comprising a template strand, a complementary strand and a double-stranded end; and (d) ligating a second adapter comprising a hairpin to the double-stranded end of the extended polynucleotides by double-stranded ligation to obtain library polynucleotides;

Not encompassed by the wording of the claims, some embodiments also include converting conversion-sensitive cytosine residues of the library polynucleotides to another base residue to obtain converted polynucleotides. In some embodiments, the converting comprises bisulfite conversion. In some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, in some embodiments, the first adapter comprises conversion-resistant cytosine analogs.

Not encompassed by the wording of the claims, in some embodiments, the first tailed primer comprises a sequencing primer binding site, an amplification primer binding site, and/or a target site for a capture probe.

Not encompassed by the wording of the claims, some embodiments also include amplifying the converted polynucleotides with second tailed primers to obtain library polynucleotides. In some embodiments, the second tailed primers comprise a sequencing primer binding site, an amplification primer binding site, and/or a target site for a capture probe.

Not encompassed by the wording of the claims, some embodiments also include sequencing the converted polynucleotides. Some embodiments also include aligning sequences of the converted polynucleotides with a reference sequence. Some embodiments also include aligning a sequence of a template strand with a sequence of a complementary strand. Some embodiments also include mapping a methylated cytosine residue on a sequence of a converted polynucleotide or a reference sequence.

Not encompassed by the wording of the claims, in some embodiments, the plurality of double-stranded template nucleic acids comprises genomic DNA or cell-free DNA.

Some embodiments of the methods and compositions provided herein include a method of preparing a nucleic acid library, comprising: (a) obtaining a plurality of double-stranded template nucleic acids by (i) contacting double stranded DNA with a plurality of transposomes comprising a first adapter to obtain DNA fragments, wherein the first adapter comprises a hairpin and cleavable site, and (ii) end-filling each DNA fragment, wherein each end of the double-stranded template nucleic acids comprises the first adapter; (b) denaturing the double-stranded template nucleic acids to obtain single-stranded template nucleic acids comprising the cleavable sites; (c) cleaving the cleavable sites to remove a portion of the first adapter from the single-stranded template nucleic acids such that an end of the cleaved single-stranded template nucleic acids comprises a hairpin; and (d) extending the hairpins of the cleaved single-stranded template nucleic acids in the presence of a conversion-resistant cytosine analog to obtain extended hairpins comprising a template strand and a complementary strand.

Some embodiments also include converting conversion-sensitive cytosine residues of the library polynucleotides to another base residue to obtain converted polynucleotides. In some embodiments, the converting comprises bisulfite conversion. In some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, in some embodiments, the first adapter comprises conversion-resistant cytosine analogs. In some embodiments, the first adapter comprises a sequencing primer binding site, an amplification primer binding site, and/or a target site for a capture probe.

Not encompassed by the wording of the claims, in some embodiments, (c) comprises contacting the cleavable site with an enzyme selected from a uracil DNA glycosylase (UDG), a DNA glycosylase-lyase, an RNase H, or a combination thereof.

Not encompassed by the wording of the claims, in some embodiments, the second tailed primers comprise a sequencing primer binding site, an amplification primer binding site, and/or a target site for a capture probe.

Some embodiments also further include sequencing the converted polynucleotides. optionally, further comprising:
(i) aligning sequences of the converted polynucleotides with a reference sequence;
(ii) aligning a sequence of a template strand with a sequence of a complementary strand; and/or
(iii) mapping a methylated cytosine residue on a sequence of a converted polynucleotide or a reference sequence.

Not encompassed by the wording of the claims, in some embodiments, the plurality of double-stranded template nucleic acids comprises genomic DNA or cell-free DNA.

### BRIEF DESCRIPTION OF THE DRAWINGS

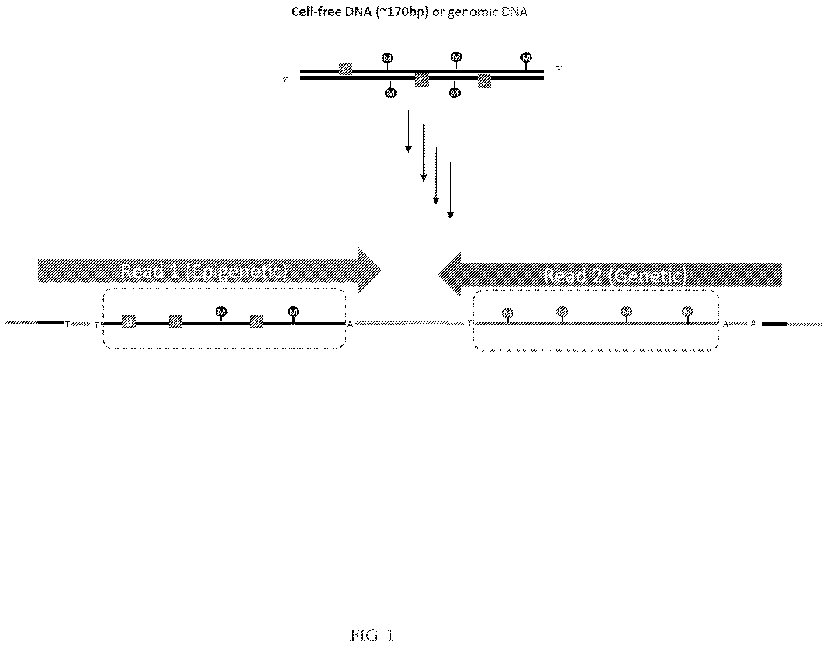

FIG. 1 depicts a scheme in which a converted polynucleotide is prepared from genomic DNA or cell-free DNA, and a converted 'epigenetic' portion of the converted polynucleotide is sequenced prior to a 'genetic' portion of the converted polynucleotide.

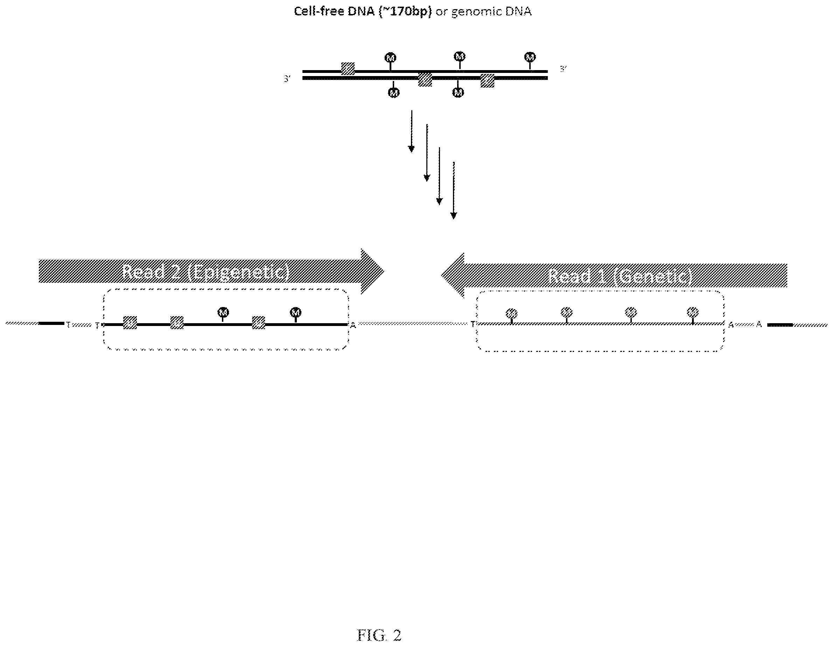

FIG. 2 depicts a scheme in which a converted polynucleotide is prepared from genomic DNA or cell-free DNA, and a 'genetic' portion of the converted polynucleotide is sequenced prior to an 'epigenetic' portion of the converted polynucleotide.

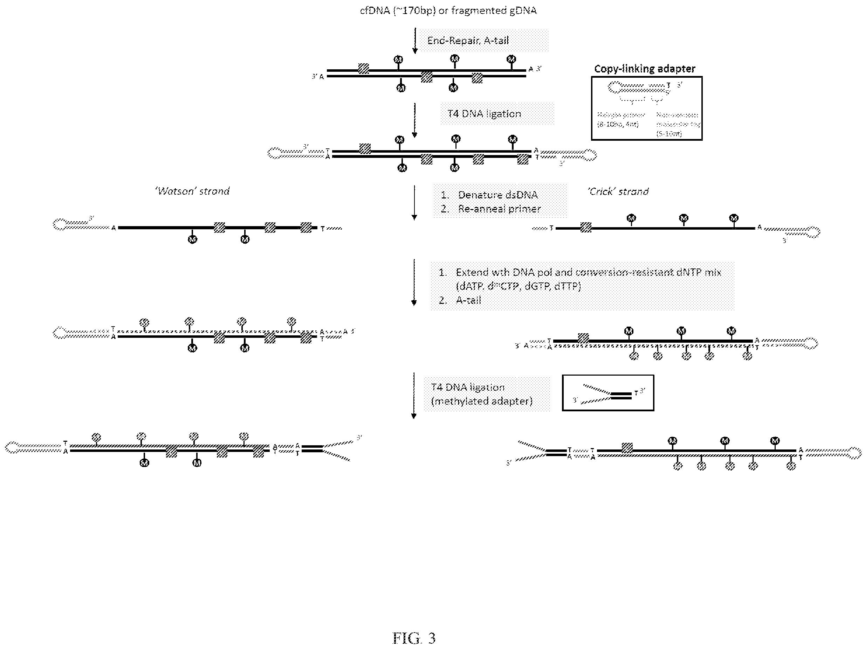

FIG. 3 depicts a workflow for the generation of polynucleotides in which a copy-linking adapter comprising a hairpin, a nick and a non-random molecular tag is ligated to a double-stranded DNA fragment by double-stranded ligation, the double-stranded DNA is denatured, the hairpin is extended in the presence of conversion-resistant nucleotides, and a Y-adapter is ligated to a double-stranded end of the extended hairpin.

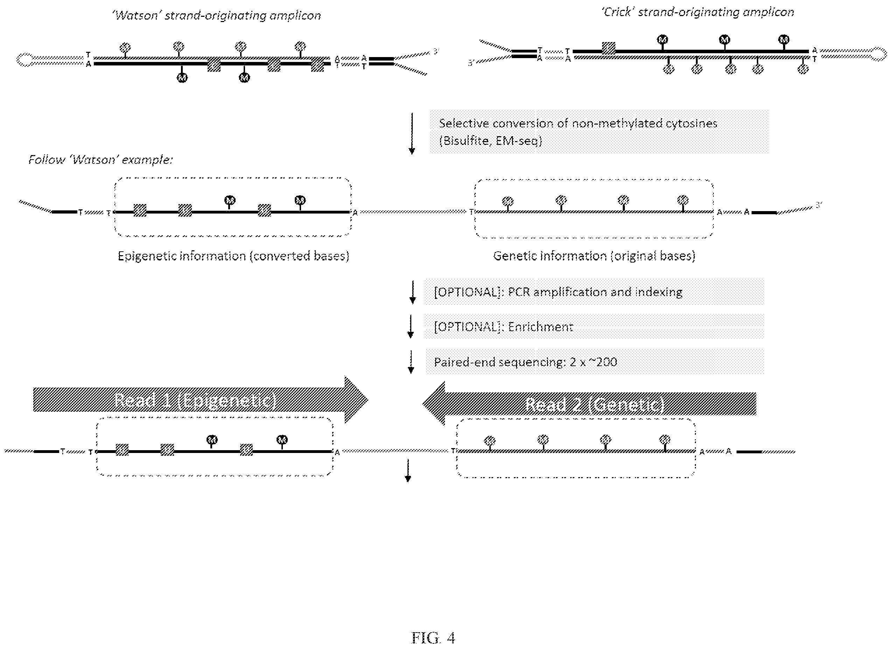

FIG. 4 depicts further processing workflow of the product shown in FIG. 3 in which non-methylated cytosines are converted, such as selectively converted to other nucleotides to obtain converted polynucleotides and the converted polynucleotides are further processed, such as amplified, indexed and/or enriched, and sequenced.

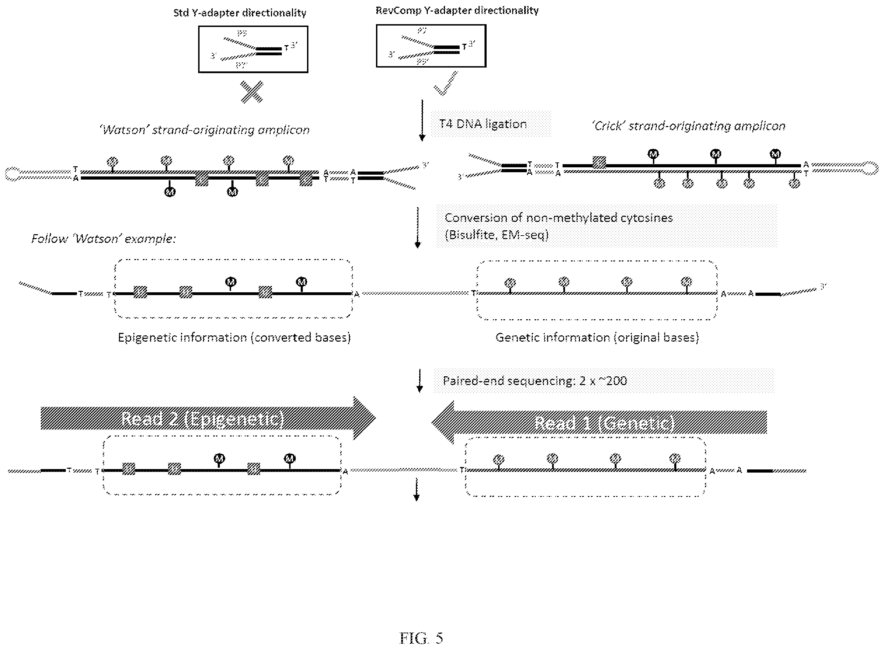

FIG. 5 depicts a portion of a workflow in which an alternative Y-shaped adapter is used to modify the order in which a 'genetic' portion of the converted polynucleotide is sequenced prior to an 'epigenetic' portion of the converted polynucleotide.

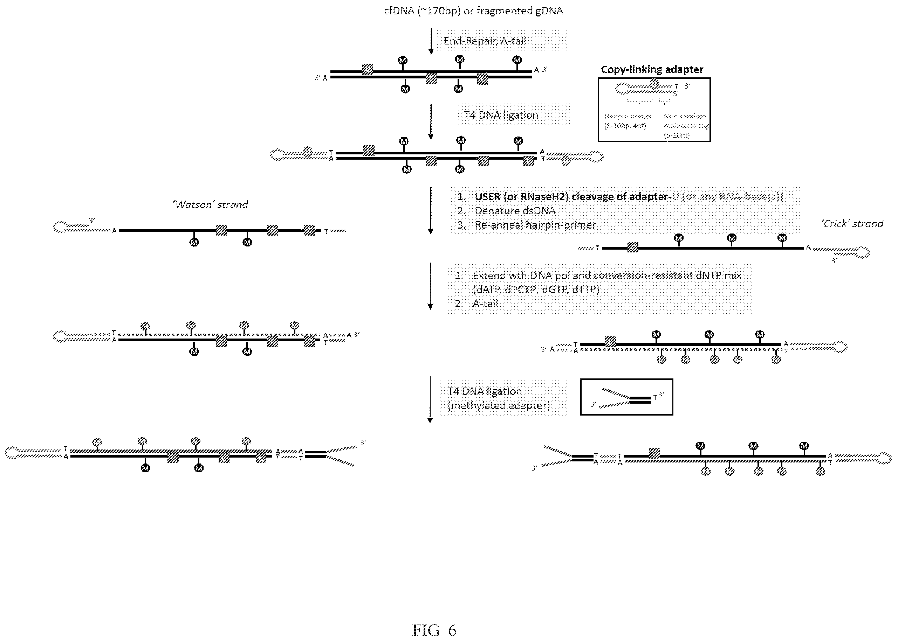

FIG. 6 depicts a workflow with an adapter comprising a cleavable site, for the generation of polynucleotides in which a copy-linking adapter comprising a hairpin, a cleavable site (a uracil) and a non-random molecular tag is ligated to a double-stranded DNA fragment by double-stranded ligation. The site is then cleaved and the double-stranded DNA is denatured, the hairpin is extended in the presence of conversion-resistant nucleotides, and a Y-adapter is ligated to a double-stranded end of the extended hairpin. The products can undergo further processing, such as by methods and steps depicted in FIG. 4.

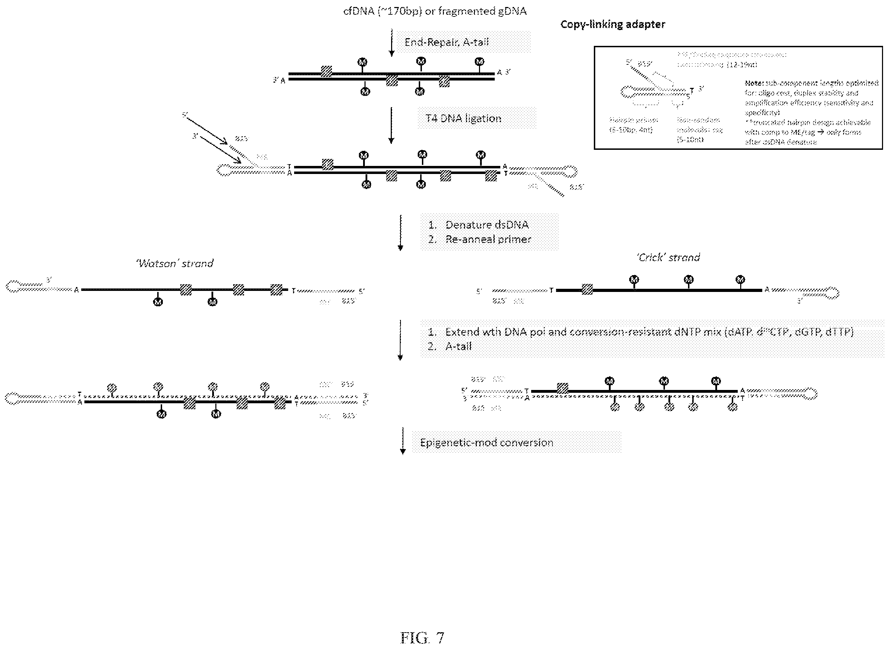

FIG. 7 depicts a workflow comprising a single ligation step for the generation of polynucleotides in which a copy-linking adapter comprising is ligated to a double-stranded DNA fragment by double-stranded ligation; the double-stranded DNA is denatured; the hairpin is extended in the presence of conversion-resistant nucleotides. The copy-linking adapter comprises a first adapter strand and second adapter strand, wherein the first adapter strand comprises a hairpin and a double-stranded region formed between a 5' end of the first adapter strand and a 3' end of the second adapter strand, wherein a 5' end of the second adapter strand is single-stranded.

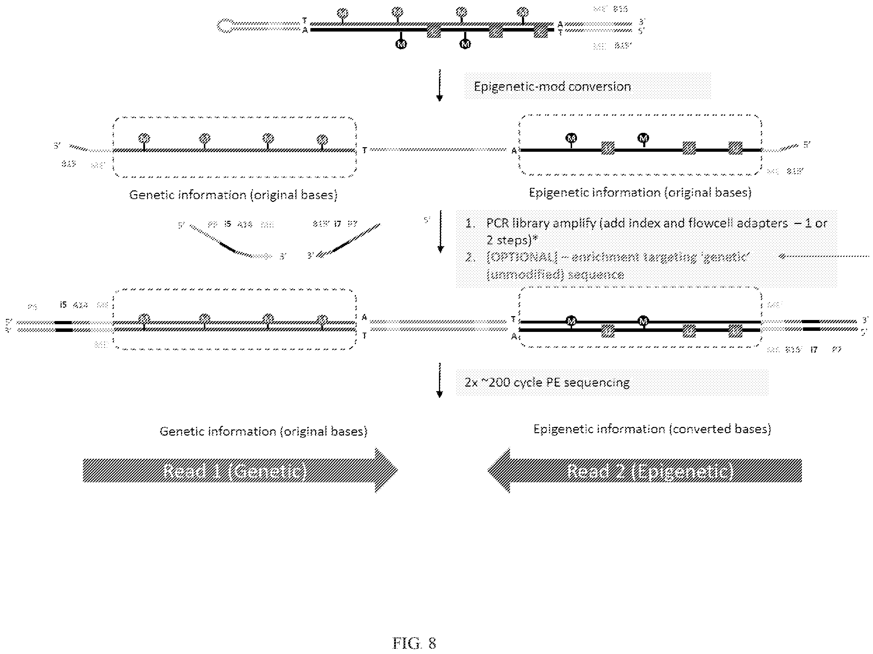

FIG. 8 depicts further processing of the product shown in FIG. 7 in which non-methylated cytosines are selectively converted to other nucleotides to obtain converted polynucleotides; and the converted polynucleotides are amplified using tailed primers containing P5 or P7 sequences and index sequences (i5 or i7), and sequenced.

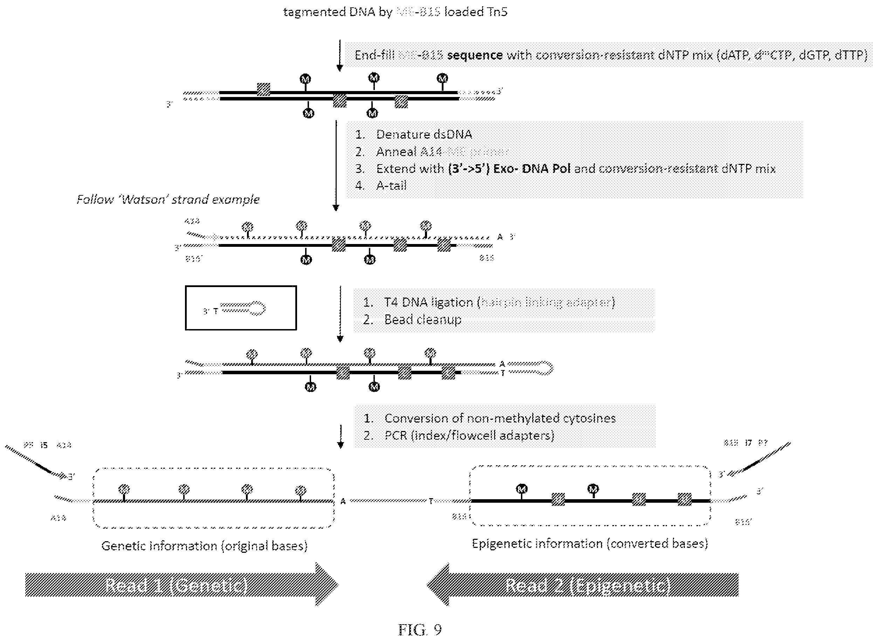

FIG. 9 depicts a workflow comprising use of tagmented DNA with a ligation step for the generation of polynucleotides. In the workflow, the tagmented DNA fragments comprise adapters; the double-stranded DNA is denatured; a tailed primer is annealed to the single-stranded polynucleotides and extended in the presence of conversion-resistant nucleotides to obtain extended polynucleotides comprising a template strand, a complementary strand and a double-stranded end; a second adapter comprising a hairpin is ligated to the double-stranded end of the extended polynucleotides by double-stranded ligation to obtain library polynucleotides; and the library polynucleotides are converted and undergo further processing, such as the addition of indexes and/or flowcell adapters by amplification with tailed primers.

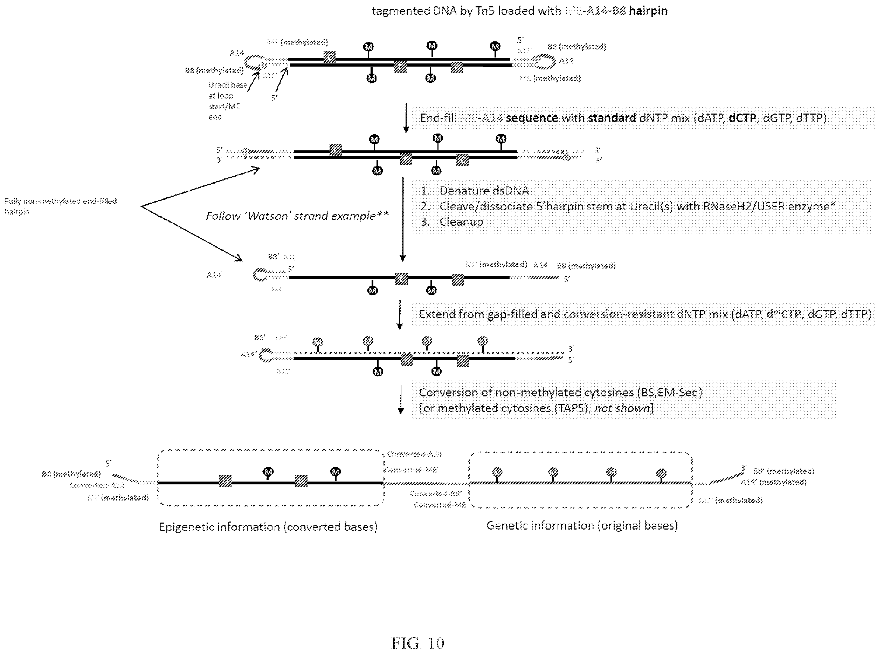

FIG. 10 depicts a workflow comprising use of tagmented DNA with hairpin adapters for the generation of polynucleotides. In the workflow, the tagmented DNA fragments comprise adapters comprising a sequence capable of forming a hairpin and a cleavable site (U nucleotide). The fragments are end-filled, and the cleavable site is cleaved. The hairpin of the adapter is extended in the presence of conversion-resistant nucleotides to obtain extended polynucleotides. The extended hairpins are converted to obtain converted polynucleotides.

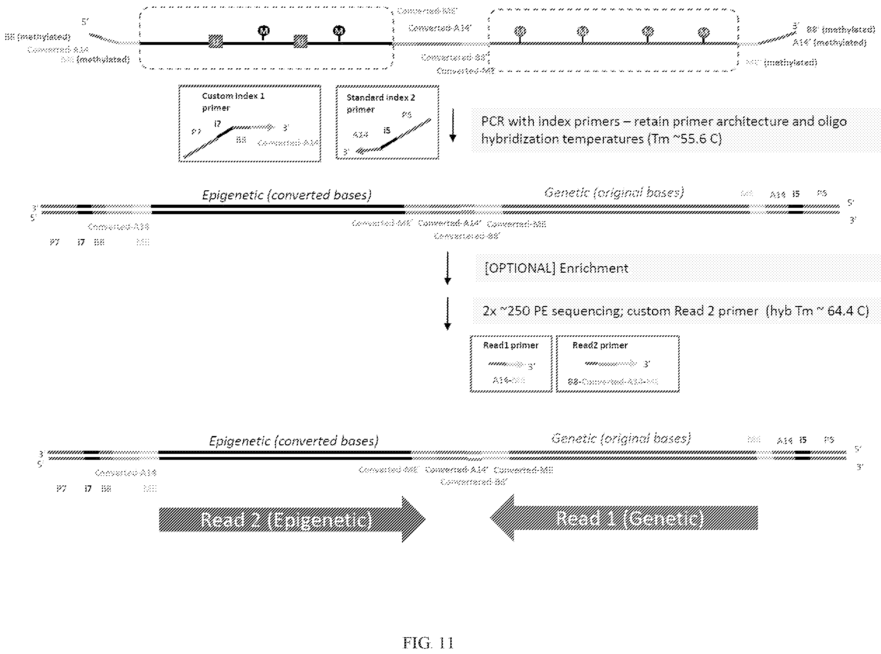

FIG. 11 depicts further processing of the converted polynucleotides depicted in FIG. 10 in which flow cell adapter (e.g., P5, P7 sequences) and indexes (e.g., i5 and i7) are added to the converted polynucleotides by amplification with tailed primers, and the products are sequenced.

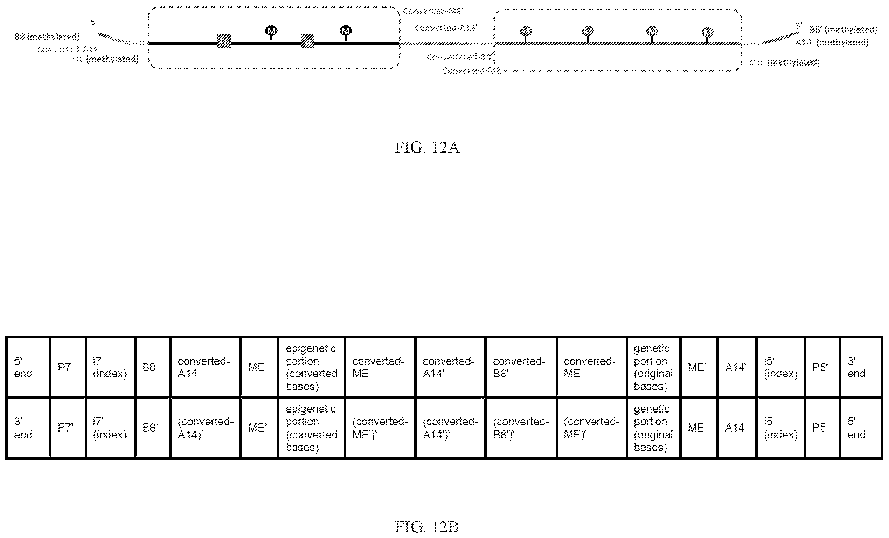

FIG. 12A is a graphical image which depicts a single-stranded converted polynucleotide.
FIG. 12B is a block chart which graphically depicts a double stranded product of amplification of the single-stranded converted polynucleotide with tailed primers adding P7, P5, and index (i7, i5) sequences.

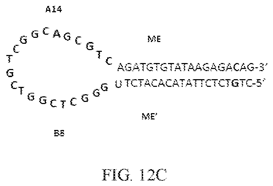

FIG. 12C depicts an example first adapter comprising a hairpin, a neck, and a cleavable site comprising a uracil nucleotide useful for workflows described herein.

### DETAILED DESCRIPTION

Some embodiments of the methods and compositions provided herein relate to preparation of libraries to determine methylation status of a nucleic acid. Some such embodiments include denaturing a double-stranded polynucleotide to obtain a first single-strand containing methylated and non-methylated cytosines (conversion-sensitive); copying the first single-strand in the presence of conversion-resistant nucleotides to obtain a second single-strand; and selectively converting conversion-sensitive cytosines in the first strand to another nucleotide. Some embodiments provided herein relate to efficient preparation of libraries in which the first stand and second strand remain physically linked to one another.

Some embodiments include the use of a double-stranded ligation with hairpin adapters and Y-shaped adapters. Some embodiments include the use of a single ligation step with modified hairpin adapters. Some embodiments include the use of transposomes to generate nucleic acid fragments comprising adapters at each end of the fragments, and the use of a hairpin adapter. Some embodiments include the use of transposomes to generate nucleic acid fragments comprising adapters at each end of the fragments in which the adapters are capable of forming hairpins. Some embodiments include the use of adapters comprising tags such that a sequence derived from a template strand of a nucleic acid of a sample can be matched with a sequence derived from the complementary strand of the nucleic acid of the sample.

The methylation status of nucleic acids is important information that is useful in many biological assays and studies. Very often, it is of particular interest to identify patterns of methylation at specific regions in the genome. Also, it is often of particular interest to identify the methylation status of specific CpG dinucleotides.

The methylation level and pattern of a locus in a nucleic acid sample can be determined using any of a variety of methods described herein and capable of distinguishing presence or absence of a methyl group on a nucleotide base of the nucleic acid. In the case of DNA, methylation, when present, typically occurs as 5-methylcytosine (5-mCyt) in CpG dinucleotides. Methylation of CpG dinucleotide sequences or other methylated motifs in DNA can be measured using any of a variety of techniques used in the art for the analysis of specific CpG dinucleotide methylation status.

A commonly-used method of determining the methylation level and/or pattern of DNA requires methylation status-dependent conversion of cytosine in order to distinguish between methylated and non-methylated CpG dinucleotide sequences. For example, methylation of CpG dinucleotide sequences can be measured by employing cytosine conversion-based technologies, which rely on methylation status-dependent chemical modification of CpG sequences within isolated genomic DNA, or fragments thereof, followed by DNA sequence analysis. Chemical reagents that are able to distinguish between methylated and non-methylated CpG dinucleotide sequences include hydrazine, which cleaves the nucleic acid, and bisulfite treatment. Bisulfite treatment followed by alkaline hydrolysis specifically converts non-methylated cytosine to uracil, leaving 5-methylcytosine unmodified as described by Olek A., Nucleic Acids Res. 24:5064-6, 1996 or Frommer et al., Proc. Natl. Acad. Sci. USA 89:1827-1831 (1992).. The bisulfite-treated DNA can subsequently be analyzed by conventional molecular techniques, such as PCR amplification, sequencing, and detection comprising oligonucleotide hybridization.

Some embodiments of the invention include to use of bisulfite conversion conditions and bisulfite-resistant cytosine analogs. One consequence of bisulfite-mediated deamination of cytosine is that the bisulfite treated cytosine is converted to uracil, which reduces the complexity of the genome. Specifically, a typical 4-base genome (A,T,C,G) is essentially reduced to a 3-base genome (A,T,G) because uracil is read as thymine during downstream analysis techniques such as PCR and sequencing reactions. Thus, the only cytosines present are those that were methylated prior to bisulfite conversion. Because the complexity of the genome is reduced, standard methods for comparing and/or aligning a bisulfite-converted sequence to the pre-conversion genome can be cumbersome and, in some cases, ineffective. For example, problems may arise when aligning converted fragments to the genome, especially when using short sequences. Accordingly, embodiments of the invention facilitate identification of the genomic context of bisulfite converted DNA as described below.

Provided herein are methods and compositions that ameliorate problems that arise from the reduced genomic complexity after bisulfite conversion of nucleic acids. For example, some embodiments described herein relate to methods of sequencing nucleic acids and determining the methylation level and/or pattern of the nucleic acids. Other embodiments relate to nucleic acid pairs, arrays and methods of making arrays useful for determining the methylation level and/or pattern of nucleic acids. Using the methods and/or compositions described herein, complexity of the target nucleic acids is preserved by keeping track of complementary strands after the strands have been subjected to bisulfite conversion of nucleic acids.

Some embodiments provided herein relate to making methylated copies of the target nucleic acids prior to bisulfite conversion. The methylated copies can then be sequenced and compared or aligned to the converted target nucleic acids. The methods provided herein are particularly useful in multiplex formats wherein several nucleic acids having different sequences and/or different methylation patterns are assayed in a common sample or pool. Thus, embodiments of the methods set forth herein can provide the advantage of avoiding the need for separation of different sequences into separate vessels during one or more steps of a methylation detection assay. For example, as set forth in further detail below in regard to particular embodiments, several pairs of nucleic acids can be treated with bisulfite in a common pool and the differences in methylation status for individual nucleic acids from the pool can then be determined.

Although many of the methods and compositions disclosed herein are exemplified or described in connection with DNA, it will be appreciated that these methods and compositions can be used with or include other nucleic acids. Furthermore, it will be understood that methods and compositions described in the context single nucleic acid molecules can also relate to methods and compositions that include or comprise a plurality of the same, similar and/or different nucleic acids. Such embodiments are often referred to as multiplex embodiments. In these multiplex embodiments, the methods are performed using and the compositions comprise a population of nucleic acids. In some embodiments, the population of nucleic acids may be divided into one or more sub-populations.

Certain embodiments provided herein include aspects disclosed in U.S. Pat. No. 8,895,268..

### Definitions

As used herein, reference to determining the methylation status and like terms refers to at least one or more of the following: 1) determining the level or amount of cytosine methylation in a sample, 2) determining the position of methylated cytosine residues within a sequence, 3) determining the pattern of methylated cytosine in a sequence, and/or 4) determining the whole sequence including the specific position and identity of methylated residues in the context of the sequence.

As used herein, "nucleic acid polymerase" or "polymerase" refers to an enzyme that catalyzes the polymerization of nucleoside triphosphates, and encompasses DNA polymerases, RNA polymerases, reverse transcriptases and the like. Generally, the enzyme will initiate synthesis at the 3'-end of the primer annealed to a template sequence, and will proceed in the 5'-direction along the template, and if possessing a 5' to 3' nuclease activity, it may hydrolyze intervening, annealed probe to release both labeled and unlabeled probe fragments, until synthesis terminates.

As used herein, the term "DNA polymerase" refers to an enzyme which catalyzes the synthesis of DNA. It uses a one strand of the DNA duplex as a template. For example, templates may include, but are not limited to, single-stranded DNA, partially duplexed DNA and nicked double-stranded DNA. The polymerase can generate a new strand from primers hybridized to the template. As presented herein, an oligonucleotide primer is used which has a free 3'-OH group. The polymerase then copies the template in the 5' to 3' direction provided that sufficient quantities of free nucleotides, such as dATP, dGTP, dCTP, 5-methyl dCTP and dTTP are present. Examples of DNA polymerases include, but are not limited to, E. coli DNA polymerase I, the large proteolytic fragment of E. coli DNA polymerase I, commonly known as "Klenow" polymerase, "Taq" polymerase, T7 polymerase, Bst DNA polymerase, T4 polymerase, T5 polymerase, reverse transcriptase, exo-BCA polymerase, Thermus thermophilus (Tth) DNA polymerase, Bacillus stearothermophilus DNA polymerase, Thermococcus litoralis DNA polymerase, Thermus aquaticus (Taq) DNA polymerase and Pyrococcus furiosus (PfU) DNA polymerase.

In embodiments of the present invention, the DNA polymerase copies the template in the 5' to 3' direction in the presence of 5-methyl dCTP, or any other suitable nucleotide which is resistant to cytosine conversion such as bisulfate conversion.

As used herein, "converted," when used in reference to a nucleic acid or portion thereof, refers to nucleic acid or a portion thereof which has been treated under conditions sufficient to convert cytosine to another base. As used herein, "bisulfite-converted", "bisulfite-treated" and like terms, when used in reference to a nucleic acid or portion thereof, refer to nucleic acid or a portion thereof which has been treated with sodium bisulfite under conditions sufficient to convert cytosine to uracil. Thus, for example, in some embodiments, template nucleic acid will have at least one cytosine residue that is not methylated and which is converted to uracil by bisulfite treatment. However, the template nucleic acid need not comprise a non-methylated cytosine, either because all cytosines are methylated or because no cytosine residues are present in the template nucleic acid.

As used herein, "non-converted," when used in reference to a nucleic acid or portion thereof, refers to a nucleic acid or portion thereof where one or more of the cytosines, if present, are not converted to another base, such as uracil, after conversion treatment, such as treatment with sodium bisulfite. Thus, for example, a non-converted complementary copy is a nucleic acid that comprises one or more bisulfite-resistant cytosine analogs that prevent the conversion of cytosine to uracil.

As used herein, "conversion-resistant cytosine analog" and like terms refer to cytosine analogs which, when incorporated into DNA, RNA, or other nucleic acid polymers, are refractory to being changed into another base under conditions where cytosine is converted into the other base. As used herein, "bisulfite-resistant cytosine analog" and like terms refer to cytosine analogs which, when incorporated into DNA, RNA, or other nucleic acid polymers, are refractory to deamination caused in reactions with sodium bisulfite. Bisulfite-resistant cytosine analogs are known in the art and can include any cytosine analog with the above-described property. Thus, for example, 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP, or any other bisulfite-resistant nucleotides comprising a cytosine analog can be used in the present embodiments as bisulfite-resistant cytosine analogs. Typically, the bisulfite-resistant cytosine analog is 5-methyl dCTP. Although 5-methyl dCTP and 5-methylcytosine are referred to in the description, examples and figures, it will be readily understood that any suitable bisulfite-resistant cytosine analog can be used in such embodiments.

In some embodiments, "cytosine" refers to nucleotides, nucleosides, nucleotide triphospates and the like which include cytosine (i.e., 4-amino-3H-pyrimidin-2-one) as the base. Thus, for example, where embodiments describe replacing cytosine with a bisulfite-resistant cytosine analog such as 5-methyl cytosine, it will be understood that the term cytosine does not include cytosine residues that are methylated at the 5-position of the cytosine base, unless specifically indicated to the contrary. In some embodiments, the term cytosine can refer to a base structure that is common between cytosine and cytosine analogs, including bisulfite-resistant cytosine analogs, as described in detail herein.

In some embodiments, 5-methyl dCTP replaces all cytosines that complement guanine positions in a complementary copy. In some embodiments, 5-methyl dCTP replaces at least one cytosine that complements a guanine position in the complementary copy. In other embodiments, 5-methyl dCTP replaces at least 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, 5% or at least 1% of the cytosines that complement guanine positions in the complementary copy. Upon treatment of the complementary copy with sodium bisulfate, those methylated cytosines in the complementary copy are refractory to the deamination reaction, and therefore the genomic complexity is maintained.

As used herein "template nucleic acid" refers to that strand of a polynucleotide from which a complementary polynucleotide strand can be hybridized or synthesized by a nucleic acid polymerase, for example, in a primer extension reaction. In some embodiments, the template nucleic acid is a template DNA.

In embodiments disclosed herein, a template nucleic acid is provided and a complementary copy of the template nucleic acid is generated or provided. The template nucleic acid can be either a single DNA strand or one or both of the single strands in a double-stranded molecule. In embodiments where the template nucleic acid is single stranded, the complementary copy is generated by extending an oligonucleotide primer with a nucleic acid polymerase such that a complementary copy of some or part of the template strand is extended in the 3' direction of the oligonucleotide primer. In a preferred embodiment, the template nucleic acid comprises a template DNA.

In embodiments where the nucleic acid is double-stranded, one or both strands may serve as the template strand for nucleic acid polymerase. For example, where one strand (the "sense" strand) serves as template, a complementary copy is generated which is complementary to the sense strand. Likewise, where the antisense strand serves as template, a complementary copy is generated which is complementary to the antisense strand. Where both strands serve as template, a separate complementary copy is generated for each of the sense and antisense strands. In a preferred embodiment, each strand of a double-stranded DNA molecule is a template nucleic acid.

As used herein, the term "complementary" refers to nucleic acid sequences that are capable of forming Watson-Crick base-pairs. For example, a complementary sequence of a first sequence is a sequence which is capable of forming Watson-Crick base-pairs with the first sequence. The term "complementary" does not necessarily mean that a sequence is complementary to the full-length of its complementary strand, but the term can mean that the sequence is complementary to a portion thereof. Thus, in some embodiments, complementarity encompasses sequences that are complementary along the entire length of the sequence or a portion thereof. For example, two sequences can be complementary to each other along at least 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, or at least 200 consecutive nucleotides. Also, as used herein, a statement that one sequence is complementary to another sequence also encompasses situations in which the two sequences have some mismatches. For example, complementary sequences can include sequences that are complementary along at least 70%, 75%, 80%, 85%, 90%, 91%, 92%, 93%, 94%, 95%, 96%, 97%, 98%, 99%, or 100% of the length of the sequence. Here, the term "sequence" encompasses, but is not limited to, nucleic acid sequences, polynucleotides, oligonucleotides, probes, primers, primer-specific regions, and target-specific regions. Despite the mismatches, the two sequences should have the ability to selectively hybridize to one another under appropriate conditions.

A first nucleic acid strand that is converted, for example using bisulfate treatment, can have conversion induced non-complementarity with a second strand to which it was previously complementary. For example, cytosines in a first nucleic acid strand may be converted to uracils, such that positions in the first strand that formerly contained cytosines capable of forming Watson-Crick base pairs with guanines at comparable positions in the second, complementary strand are no longer capable of doing so. Nevertheless, for ease of identification, the converted nucleic acid strand may be identified with respect to complementarity of the previous, non-converted first strand to the second strand.

As used herein, the terms "pairing" or "paired" and the like refer to methods used to match a nucleic acid template with its corresponding complementary copy. For example, pairing can be accomplished via a physical tether between the complementary copy and the bisulfite-converted template nucleic acid. Additionally or alternatively, pairing can be accomplished via tag molecules which identify the complementary copy and the bisulfite-converted template nucleic acid as members of a nucleic acid pair. Tag molecules are useful, for example, where the nucleic acid template and the complementary copy are not physically tethered together. Thus, through the use of tag molecules, the two paired members are matched and recognized as members of a pair. The use of covalent tethers and tag molecules are described in further detail below. Also, it will be appreciated that the term "pairing" is not limited to an action or step that must occur at a particular point in the processes described herein. Pairing of nucleic acid sequences can occur at any point in the process that would allow information the information present in a complementary strand to be associated with the information present in a corresponding template strand. As such, when used as a noun, the term "pairing" can refer to any two or more associated nucleic acid strands, whether associated physically or via other methods such as tagging or labeling, that are present at any step of a process described herein or that are present in any of the compositions described herein.

### Preparing nucleic acid libraries

Some embodiments of the methods and compositions provided herein include preparing nucleic acid libraries. Such libraries can be useful to determine the location of methylated residues in a nucleic acid.

Some embodiments include methods of preparing a nucleic acid library, comprising (a) obtaining a plurality of double-stranded template nucleic acids, and a first adapter comprising a hairpin and a double-stranded region comprising a nick or a nickable site, wherein the nickable site comprises a uracil or a ribonucleotide; (b) ligating the first adapter to each end of the double-stranded template nucleic acids by double-stranded ligation; (c) denaturing the double-stranded template nucleic acids to obtain single-stranded template nucleic acids comprising a hairpin; (d) extending the hairpin in the presence of a conversion-resistant cytosine analog to obtain extended hairpins comprising a template strand and a complementary strand; and (e) ligating a Y-adapter to an end of the extended hairpins by double-stranded ligation to obtain library polynucleotides. Not encompassed by the wording of the claims, in some embodiments, the plurality of double-stranded template nucleic acids comprises genomic DNA or cell-free DNA.

Not encompassed by the wording of the claims, some embodiments also include converting conversion-sensitive cytosine residues of the library polynucleotides to another base residue to obtain converted polynucleotides. In some embodiments, the converting comprises bisulfite conversion. In some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP.

Not encompassed by the wording of the claims, in some embodiments, the double-stranded region of the first adapter comprises the nick.

In some embodiments, the double-stranded region of the first adapter comprises the nickable site. In some embodiments, (c) comprises contacting the nickable site with an enzyme prior to the denaturing, wherein the enzyme is selected from a uracil DNA glycosylase (UDG), a DNA glycosylase-lyase, an RNase H, or a combination thereof.

In some embodiments, the Y-adapter comprises a double-stranded portion and a non-complementary portion comprising a first single strand and a second single strand. In some embodiments, the first single strand and/or the second single strand comprise a sequencing primer binding site, an amplification primer binding site, and/or a target site for a capture probe.

Not encompassed by the wording of the claims, in some embodiments, the double-stranded region of the first adapter comprises a tag sequence. In some embodiments, (a) comprises obtaining a plurality of the first adapter, wherein the tag sequences of the plurality of the first adapter are different from one another. Some embodiments also include identifying a first sequence of a converted polynucleotide and a second sequence of a converted polynucleotide comprising the same tag sequence by comparing tag sequences of the library polynucleotides, thereby identifying a first sequence of a converted polynucleotide and a second sequence of a converted polynucleotide derived from the same double-stranded template nucleic acid.

Not encompassed by the wording of the claims, some embodiments also include a method comprising (i) amplifying the library polynucleotides or converted polynucleotides; and/or (ii) adding indexes to the library polynucleotides or converted polynucleotides.

Some embodiments also include sequencing the converted polynucleotides. Some embodiments also include analyzing the sequence data. For example, further comprising:
(i) aligning sequences of the converted polynucleotides with a reference sequence;
(ii) aligning a sequence of a template strand with a sequence of a complementary strand; and/or
(iii) mapping a methylated cytosine residue on a sequence of a converted polynucleotide or a reference sequence.

An example workflow is depicted in FIG. 3 and FIG. 4. For example, cell-free DNA of about 170 bp or fragmented genomic DNA is end-repaired and A-tailed to obtain double-stranded template nucleic acids. First adapters, such as copy-linking adapters, are ligated to each end of the double-stranded template nucleic acids by double-stranded ligation using a T4 DNA ligase. The first adapter includes a hairpin of about 8-10 bp in the neck and 4 nucleotides in the loop, and a double-stranded portion of about 5-10 nucleotides. The double-stranded portion of the first adapter can include a tag sequence, such as a non-random molecular tag that can be used to track products, such as nucleic acids and sequences derived from a particular template double-stranded template nucleic acid. Also, double-stranded ligation has increased efficiency over methods that include single-stranded ligation.

In some embodiments, the first adapter comprises a nick in a single-strand between the hairpin and the double-stranded portion. The nicks in each first adapter ligated to a double stranded template nucleic acid allow the double stranded template to be denatured to obtain single-stranded template nucleic acids, such as a Watson strand and a Crick strand of the double stranded template nucleic acid. The single-stranded template nucleic acids comprise the hairpin of the first adapter and a single-strand of the double-stranded portion of the first adapter. The hairpin in each single-stranded template nucleic acid can be extended to obtain an extended hairpin comprising the single-stranded template (template strand), and a complement of the single-stranded template (complementary strand). The extension is performed in the presence of conversion-resistant nucleotides, including conversion-resistant cytosine analogs. The double-stranded end of the extended hairpin can be A-tailed and a Y-adapter can be ligated to the double-stranded end of the extended hairpin by double-stranded ligation with a T4 DNA ligase to obtain library polynucleotides. The Y-adapter is double-stranded and include a portion in which the strands are complementary to one another, and a portion in which the strands are not complementary to one another. Each strand of the non-complementary portion of the Y-adapter can include sequencing primer binding sites, such as P5, P5', P7, P7' sequencing primer binding sites, amplification primer binding sites, and/or capture probe binding sites. The library polynucleotides can undergo selective conversion of non-methylated cytosine residues, such as bisulfite conversion to obtain converted polynucleotides. After conversion, the strands of the extended hairpins are typically no longer complementary to one another and form a single-stranded molecule. The single-stranded molecule can be further processed via the sequences provided by the Y-adapters, for example, amplifying and/or sequencing. The single-stranded molecule can have additional tags, such as indexes, added via extension and/or amplification using tailed primers. The sequences of the single-stranded molecules can be determined and analyzed. For example, sequences can be aligned to a reference sequence, such as a reference genome. An 'epigenetic' read of a single-stranded molecule can be aligned with a 'genetic' read of the single-stranded molecule. Sequences derived from the Watson strand and the Crick strand of a double-stranded template nucleic acid can be used to construct higher quality consensus sequences.

In some embodiments, the order an 'epigenetic' read of a single-stranded molecule and a 'genetic' read of the single-stranded molecule are obtained can be modified by modifying the Y-adapter and/or sequencing primers. An example workflow is depicted in FIG. 5. In some embodiments, the 'genetic' read of a single-stranded molecule has a higher complexity than an 'epigenetic' read of a single-stranded molecule because certain conversion-sensitive nucleotides would have been converted to other nucleotides in the 'epigenetic' read. Therefore, modifying the Y-adapter to position high complexity (unmodified) sequence in a Read 1 (cluster generation) can facilitate high-quality sequencing data (from epigenetic-converted sequence libraries).

An additional workflow is depicted in FIG. 6. For example, cell-free DNA of about 170 bp, or fragmented genomic DNA, is end-repaired and A-tailed to obtain double-stranded template nucleic acids. First adapters, such as copy-linking adapters, are ligated to each end of the double-stranded template nucleic acids by double-stranded ligation using a T4 DNA ligase. The first adapter includes a hairpin of about 8-10 bp in the neck and 4 nucleotides in the loop, and a double-stranded portion of about 5-10 nucleotides. The double-stranded portion of the first adapter can include a tag sequence, such as a non-random molecular tag that can be used to track products, such as nucleic acids and sequences derived from a particular template double-stranded template nucleic acid. Also, double-stranded ligation has increased efficiency over methods that include single-stranded ligation.

In some embodiments, the first adapter comprises a nickable site at a location between the hairpin and the double-stranded portion. Cleavage of the nickable site creates nicks in each first adapter ligated to a double stranded template nucleic acid and allow the double stranded template to be denatured to obtain single-stranded template nucleic acids, such as a Watson strand and a Crick strand of the double stranded template nucleic acid. The single-stranded template nucleic acids comprise the hairpin of the first adapter and a single-strand of the double-stranded portion of the first adapter. The hairpin in each single-stranded template nucleic acid can be extended to obtain an extended hairpin comprising the single-stranded template (template strand), and a complement of the single-stranded template (complementary strand). The extension is performed in the presence of conversion-resistant nucleotides, including conversion-resistant cytosine analogs. The double-stranded end of the extended hairpin can be A-tailed and a Y-adapter can be ligated to the double-stranded end of the extended hairpin by double-stranded ligation with a T4 DNA ligase to obtain library polynucleotides. The Y-adapter is double-stranded and include a portion in which the strands are complementary to one another, and a portion in which the strands are not complementary to one another. Each strand of the non-complementary portion of the Y-adapter can include sequencing primer binding sites, such as P5, P5', P7, and P7' sequencing primer binding sites, amplification primer binding sites, and/or capture probe binding sites.

Not encompassed by the wording of the claims, in some embodiments, the library polynucleotides can undergo selective conversion of non-methylated cytosine residues, such as bisulfite conversion to obtain converted polynucleotides. After conversion, the strands of the extended hairpins are typically no longer complementary to one another and form a single-stranded molecule. The single-stranded molecule can be further processed via the sequences provided by the Y-adapters, for example, amplifying and/or sequencing. The single-stranded molecule can have additional tags, such as indexes, added via extension and/or amplification using tailed primers. The sequences of the single-stranded molecules can be determined and analyzed. For example, sequences can be aligned to a reference sequence, such as a reference genome. An 'epigenetic' read of a single-stranded molecule can be aligned with a 'genetic' read of the single-stranded molecule. Sequences derived from the Watson strand and the Crick strand of a double-stranded template nucleic acid can be used to construct higher quality consensus sequences.

### Single ligation methods for preparing libraries

Not encompassed by the wording of the claims, some embodiments of the methods and compositions provided herein include a method of preparing a nucleic acid library including a single ligation step. Some such embodiments include a method of preparing a nucleic acid library, comprising: (a) obtaining a plurality of double-stranded template nucleic acids, and a first adapter comprising a first adapter strand and second adapter strand, wherein the first adapter strand comprises a hairpin and a double-stranded region formed between a 5' end of the first adapter strand and a 3' end of the second adapter strand, wherein a 5' end of the second adapter strand is single-stranded; (b) ligating the first adapter to each end of the double-stranded template nucleic acids by double-stranded ligation; (c) denaturing the double-stranded template nucleic acids to obtain single-stranded template nucleic acids; and (d) extending the hairpin in the presence of a conversion-resistant cytosine analog to obtain to obtain library polynucleotides comprising a template strand and a complementary strand. In some embodiments, the plurality of double-stranded template nucleic acids comprises genomic DNA or cell-free DNA.

Not encompassed by the wording of the claims, some embodiments also include converting conversion-sensitive cytosine residues of the library polynucleotides to another base residue to obtain converted polynucleotides. In some embodiments, the converting comprises bisulfite conversion. In some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, in some embodiments, the single-stranded 5' end of the second adapter strand comprises a sequencing primer binding site, an amplification primer binding site, and/or a target site for a capture probe. In some embodiments, the tailed primers comprise a sequencing primer binding site, an amplification primer binding site, and/or a target site for a capture probe. In some embodiments, the double-stranded region of the first adapter a sequencing primer binding site, an amplification primer binding site, and/or a target site for a capture probe. In some embodiments, the double-stranded region of the first adapter comprises a tag sequence.

Not encompassed by the wording of the claims, in some embodiments, (a) comprises obtaining a plurality of the first adapter, wherein the tag sequences of the plurality of the first adapter are different from one another. Some embodiments also include identifying a first sequence of a converted polynucleotide and a second sequence of a converted polynucleotide comprising the same tag sequence by comparing tag sequences of the converted polynucleotides, thereby identifying a first sequence of a converted polynucleotide and a second sequence of a converted polynucleotide derived from the same double-stranded template nucleic acid.

Not encompassed by the wording of the claims, some embodiments also include (i) amplifying the library polynucleotides or converted polynucleotides; and/or (ii) adding indexes to the library polynucleotides or converted polynucleotides.

Not encompassed by the wording of the claims, some embodiments also include sequencing the converted polynucleotides. Some embodiments also include aligning sequences of the converted polynucleotides with a reference sequence. Some embodiments also include aligning a sequence of a template strand with a sequence of a complementary strand. Some embodiments also include mapping a methylated cytosine residue on a sequence of a converted polynucleotide or a reference sequence.

Another example workflow is depicted in FIG. 7 and FIG. 8. For example, cell-free DNA of about 170 bp or fragmented genomic DNA is end-repaired and A-tailed to obtain double-stranded template nucleic acids. First adapters, such as copy-linking adapters, are ligated to each end of the double-stranded template nucleic acids by double-stranded ligation using a T4 DNA ligase. The first adapter comprises a first adapter strand and second adapter strand, wherein the first adapter strand comprises a hairpin and a double-stranded region formed between a 5' end of the first adapter strand and a 3' end of the second adapter strand, wherein a 5' end of the second adapter strand is single-stranded. The first adapter includes a non-random molecular tag. The structure of the first adapter allows the double stranded template to be denatured to obtain single-stranded template nucleic acids, such as a Watson strand and a Crick strand of the double stranded template nucleic acid. A first end of the single-stranded template nucleic acid comprises the first adapter strand of the first adapter. A second end of the single-stranded template nucleic acid comprises the second adapter strand of the first adapter. The hairpin in each single-stranded template nucleic acid can be extended to obtain an extended hairpin comprising the single-stranded template (template strand), and a complement of the single-stranded template (complementary strand). The extension is performed in the presence of conversion-resistant nucleotides, including conversion-resistant cytosine analogs.

Not encompassed by the wording of the claims, the extended hairpin can undergo selective conversion of non-methylated cytosine residues, such as bisulfite conversion to obtain converted polynucleotides. After conversion, the strands of the extended hairpins are typically no longer complementary to one another and form a single-stranded molecule. The converted polynucleotides can be further processed via the adapter sequences, for example, amplifying and/or sequencing. The single-stranded molecule can have additional tags, such as indexes, added via extension and/or amplification using tailed primers. The sequences of the converted polynucleotides can be determined and analyzed. For example, sequences can be aligned to a reference sequence, such as a reference genome. An 'epigenetic' read of a single-stranded molecule can be aligned with a 'genetic' read of the single-stranded molecule. Sequences derived from the Watson strand and the Crick strand of a double-stranded template nucleic acid can be used to construct higher quality consensus sequences.

Not encompassed by the wording of the claims, in some embodiments, an enrichment step can be performed on the converted polynucleotides by targeting the unmodified portion of molecule. Some such embodiments include less enrichment probes and simpler design than targeting converted strand with varying methylation status and can mitigate potential enrichment hybridization biases with molecules with different methylation status.

### Tagmentation methods for preparing libraries

Not encompassed by the wording of the claims, some embodiments of the methods and compositions provided herein include a method of preparing a nucleic acid library including use of transposomes. Transposomes include a transposase and a transposon nucleic acid. In some embodiments, a transposomes can be used to fragment a nucleic acid into a plurality of fragments, wherein each fragment comprises the transposon nucleic acid located at at least one end of each fragment. In some embodiments, each fragment comprises the transposon nucleic acid located at each end of each fragment. Some such embodiments include a method of preparing a nucleic acid library, comprising: (a) obtaining a plurality of double-stranded template nucleic acids by (i) contacting double stranded DNA with a plurality of transposomes comprising a first adapter to obtain DNA fragments, and (ii) end-filling each DNA fragment, wherein each end of the double-stranded template nucleic acids comprises the first adapter; (b) denaturing the double-stranded template nucleic acids to obtain single-stranded template nucleic acids; (c) hybridizing a first tailed primer to a region at an end of the single-stranded template nucleic acids, and extending the hybridized primer in the presence of a conversion-resistant cytosine analog to obtain extended polynucleotides comprising a template strand, a complementary strand and a double-stranded end; and (d) ligating a second adapter comprising a hairpin to the double-stranded end of the extended polynucleotides by double-stranded ligation to obtain extended hairpins. In some embodiments, the plurality of double-stranded template nucleic acids comprises genomic DNA or cell-free DNA.

Not encompassed by the wording of the claims, some embodiments also include converting conversion-sensitive cytosine residues of the library polynucleotides to another base residue to obtain converted polynucleotides. In some embodiments, the converting comprises bisulfite conversion. In some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, in some embodiments, the first adapter comprises conversion-resistant cytosine analogs. In some embodiments, the first tailed primer comprises a sequencing primer binding site, an amplification primer binding site, and/or a target site for a capture probe.

Not encompassed by the wording of the claims, some embodiments also include amplifying the converted polynucleotides with second tailed primers. In some embodiments, the second tailed primers comprise a sequencing primer binding site, an amplification primer binding site, and/or a target site for a capture probe.

Not encompassed by the wording of the claims, some embodiments also include sequencing the converted polynucleotides. Some embodiments also include aligning sequences of the converted polynucleotides with a reference sequence. Some embodiments also include aligning a sequence of a template strand with a sequence of a complementary strand. Some embodiments also include mapping a methylated cytosine residue on a sequence of a converted polynucleotide or a reference sequence.

Another example workflow is depicted in FIG. 9. For example, double-stranded genomic DNA is tagmented with transposomes comprising a first adapter to obtain a plurality of nucleic acid fragments, each end of each fragment comprising the first adapter. The first adapter consists of conversion-resistant nucleotides. The ends of the fragments are end-filled. The fragments are denatured to obtain single-stranded fragments. A tailed primer is hybridized to an adapter sequence at an end of the single-stranded fragment. The tailed primer is extended in the presence of conversion resistant nucleotides to obtain extended polynucleotides comprising a template strand, a complementary strand and a double-stranded end. A second adapter comprising a hairpin is ligated to the double-stranded end of the extended polynucleotides by double-stranded ligation in the presence of T4 DNA ligase to obtain extended hairpins.

Not encompassed by the wording of the claims, the extended hairpin can undergo selective conversion of non-methylated cytosine residues, such as bisulfite conversion to obtain converted polynucleotides. After conversion, the strands of the extended hairpins are typically no longer complementary to one another and form a single-stranded molecule. The converted polynucleotides can be further processed via the first adapter sequences, for example, amplifying and/or sequencing. The single-stranded molecule can have additional tags, such as indexes, added via extension and/or amplification using tailed primers. The sequences of the converted polynucleotides can be determined and analyzed. For example, sequences can be aligned to a reference sequence, such as a reference genome. An 'epigenetic' read of a single-stranded molecule can be aligned with a 'genetic' read of the single-stranded molecule. Sequences derived from the Watson strand and the Crick strand of a double-stranded template nucleic acid can be used to construct higher quality consensus sequences.

Not encompassed by the wording of the claims, some embodiments include a method a method of preparing a nucleic acid library, comprising: (a) obtaining a plurality of double-stranded template nucleic acids by (i) contacting double stranded DNA with a plurality of transposomes comprising a first adapter to obtain DNA fragments, wherein the first adapter comprises a hairpin and cleavable site, and (ii) end-filling each DNA fragment, wherein each end of the double-stranded template nucleic acids comprises the first adapter; (b) denaturing the double-stranded template nucleic acids to obtain single-stranded template nucleic acids comprising the cleavable sites; (c) cleaving the cleavable sites to remove a portion of the first adapter from the single-stranded template nucleic acids such that an end of the cleaved single-stranded template nucleic acids comprises a hairpin; (d) extending the hairpins of the cleaved single-stranded template nucleic acids in the presence of a conversion-resistant cytosine analog to obtain library polynucleotides comprising a template strand and a complementary strand. In some embodiments, the plurality of double-stranded template nucleic acids comprises genomic DNA or cell-free DNA.

Some embodiments also include converting conversion-sensitive cytosine residues of the library polynucleotides to another base residue to obtain converted polynucleotides. In some embodiments, the converting comprises bisulfite conversion. In some embodiments, the conversion-resistant cytosine analog comprises a moiety that inhibits conversion to another base residue. In some embodiments, the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP. In some embodiments, the conversion resistant cytosine analog is 5-methyl dCTP.

Not encompassed by the wording of the claims, in some embodiments, (c) comprises contacting the cleavable site with an enzyme selected from a uracil DNA glycosylase (UDG), a DNA glycosylase-lyase, an RNase H, or a combination thereof.

Not encompassed by the wording of the claims, in some embodiments, the first adapter comprises conversion-resistant cytosine analogs. In some embodiments, the first adapter comprises a sequencing primer binding site, an amplification primer binding site, and/or a target site for a capture probe. In some embodiments, the second tailed primers comprise a sequencing primer binding site, an amplification primer binding site, and/or a target site for a capture probe.

Not encompassed by the wording of the claims, some embodiments also include sequencing the converted polynucleotides. Some embodiments also include aligning sequences of the converted polynucleotides with a reference sequence. Some embodiments also include aligning a sequence of a template strand with a sequence of a complementary strand. Some embodiments also include mapping a methylated cytosine residue on a sequence of a converted polynucleotide or a reference sequence.

Still another example workflow is depicted in FIG. 10 and FIG. 11. For example, double-stranded genomic DNA is tagmented with transposomes comprising a first adapter to obtain a plurality of nucleic acid fragments, each end of each fragment comprising the first adapter. The first adapter comprises a cleavable site and a sequence capable of forming a hairpin. The first adapter consists of conversion-resistant nucleotides which allow for epigenetic conversion of unique ends. The ends of the fragments are end-filled. The fragments are denatured to obtain single-stranded fragments. The cleavable site of a single strand is cleaved. The sequence capable of forming a hairpin of the single-stranded fragment is extended in the presence of conversion-resistant nucleotides to obtain an extended hairpin. The extended hairpin can undergo selective conversion of non-methylated cytosine residues, such as bisulfite conversion to obtain converted polynucleotides. Notably, conversion treatment generates unique adapter sequences on 5' and 3' ends, used to amplify and attach sample index and flow cell adapters.

After conversion, the strands of the extended hairpins are typically no longer complementary to one another and form a single-stranded molecule. The converted polynucleotides can be further processed via the first adapter sequences, for example, amplifying and/or sequencing. The single-stranded molecule can have additional tags, such as indexes, added via extension and/or amplification using tailed primers. The sequences of the converted polynucleotides can be determined and analyzed. For example, sequences can be aligned to a reference sequence, such as a reference genome. An 'epigenetic' read of a single-stranded molecule can be aligned with a 'genetic' read of the single-stranded molecule. Sequences derived from the Watson strand and the Crick strand of a double-stranded template nucleic acid can be used to construct higher quality consensus sequences.

### EXAMPLES

### Example 1-Indexing a prepared library

A library of converted polynucleotides is prepared by a workflow substantially similar to the workflow depicted in FIG. 10 and FIG. 11. Capture probe binding and index sequences are added to each end of the single-stranded converted polynucleotides by amplification with tailed primers. FIG. 12A depicts a single-stranded converted polynucleotide. FIG. 12B depicts a double stranded product of amplification of the single-stranded converted polynucleotide with tailed primers adding P7, P5, and index (i7, i5) sequences. Sequencing primers are used to sequence the genetic portion in a first read, and to sequence the epigenetic portion read in a second read. FIG. 12C depicts an example first adapter comprising a hairpin, a neck, and a cleavable site comprising a uracil nucleotide useful for workflows described herein. Example sequences for adapters and primers, such as those depicted in FIG. 10, FIG. 11, FIG. 12A, FIG. 12B, and FIG. 12C are listed below in TABLE 1.

**TABLE 1**

| **Feature SEQ ID NO.** | **Sequence** |
|---|---|
| A14 | TCGTCGGCAGCGTC |
| SEQ ID NO:01 | |
| A14' | GACGCTGCCGACGA |
| SEQ ID NO:02 | |
| Converted-A14 | TTGTTGGTAGTGTT |
| SEQ ID NO:03 | |
| (Converted-A14)' | AACACTACCAACAA |
| SEQ ID NO:04 | |
| Converted-A14' | GATGTTGTTGATGA |
| SEQ ID NO:05 | |
| (Converted-A14')' | TCATCAACAACATC |
| SEQ ID NO:06 | |
| ME | AGATGTGTATAAGAGACAG |
| SEQ ID NO:07 | |
| ME' | CTGTCTCTTATACACATCT |
| SEQ ID NO:08 | |
| Converted- ME | AGATGTGTATAAGAGATAG |
| SEQ ID NO:09 | |
| (Converted- ME)' | CTATCTCTTATACACATCT |
| SEQ ID NO:10 | |
| Converted- ME' | TTGTTTTTTATATATATTT |
| SEQ ID NO:11 | |
| (Converted- ME')' | AAATATATATAAAAAACAA |
| SEQ ID NO:12 | |
| B8 | GGGCTCGG |
| SEQ ID NO:13 | |
| B8' | CCGAGCCC |
| SEQ ID NO:14 | |
| Converted-B8 | GGGTTTGG |
| SEQ ID NO:15 | |
| (Converted-B8)' | CCAAACCC |
| SEQ ID NO:16 | |
| Converted-B8 | TTGAGTTT |
| SEQ ID NO:017 | |
| (Converted-B8')' | AAACTCAA |
| SEQ ID NO:18 | |
| Nextera Read1 primer (A14-ME) | TCGTCGGCAGCGTCAGATGTGTATAAGAGACAG |
| Tm = 64.5°C | |
| SEQ ID NO:19 | |
| Nextera Read2 primer (B14-ME) | GTCTCGTGGGCTCGGAGATGTGTATAAGAGACAG |
| Tm = 64.2°C | |
| SEQ ID NO:20 | |
| Custom Read2 primer (B8+convertedA14 +ME) | GGGCTCGGTTGTTGGTAGTGTTAGATGTGTATAAGAGACA G |
| Tm = 64.8°C | |
| SEQ ID NO:21 | |
| P5 | AATGATACGGCGACCACCGAGATCTACAC |
| SEQ ID NO:22 | |
| P7 | CAAGCAGAAGACGGCATACGAGAT |
| SEQ ID NO:23 | |
| Tailed index primer 1 (P7-X₇ index-B8-converted-A14) | CAAGCAGAAGACGGCATACGAGATXXXXXXXGGGCTCGG TTGTTGGTAGTGTT |
| SEQ ID NO:24 | |
| Tailed index primer 2 (P5-X₅ index-A14) | AATGATACGGCGACCACCGAGATCTACACXXXXXTCGTC GGCAGCGTC |
| SEQ ID NO:25 | |
| First adapter comprising a hairpin, neck and cleavable site | CTGTCTCTTATACACATCTUGGGCTCGGTCGTCGGCAGCG TCAGATGTGTATAAGAGACAG |
| SEQ ID NO:26 | |

## Claims

1. A method of preparing a polynucleotide library, comprising:
(a) obtaining a plurality of double-stranded template nucleic acids, and a first adapter comprising a hairpin and a double-stranded region comprising a nick or a nickable site, wherein the nickable site comprises a uracil or a ribonucleotide;
(b) ligating the first adapter to each end of the double-stranded template nucleic acids by double-stranded ligation;
(c) denaturing the double-stranded template nucleic acids to obtain single-stranded template nucleic acids comprising a hairpin;
(d) extending the hairpin in the presence of a conversion-resistant cytosine analog to obtain extended hairpins comprising a template strand and a complementary strand; and
(e) ligating a Y-adapter to an end of the extended hairpins by double-stranded ligation to obtain library polynucleotides.

2. The method of claim 1, wherein the double-stranded region of the first adapter comprises the nickable site.

3. The method of claim 1 or 2, wherein (c) comprises contacting the nickable site with an enzyme prior to the denaturing, wherein the enzyme is selected from a uracil DNA glycosylase (UDG), a DNA glycosylase-lyase, an RNase H, or a combination thereof.

4. The method of any one of claims 1-3, wherein the Y-adapter comprises a double-stranded portion and a non-complementary portion comprising a first single strand and a second single strand; optionally, wherein the first single strand and/or the second single strand comprise a sequencing primer binding site, an amplification primer binding site, and/or a target site for a capture probe.

5. The method of any one of claims 1-4, wherein the double-stranded region of the first adapter comprises a tag sequence.

6. The method of claim 5, wherein (a) comprises obtaining a plurality of the first adapter, wherein the tag sequences of the plurality of the first adapter are different from one another.

7. The method of claim 6, further comprising identifying a first sequence of a converted polynucleotide and a second sequence of a converted polynucleotide comprising the same tag sequence by comparing tag sequences of the converted polynucleotides, thereby identifying a first sequence of a converted polynucleotide and a second sequence of a library polynucleotide derived from the same double-stranded template nucleic acid.

8. The method of any one of claims 1-7, further comprising converting conversion-sensitive cytosine residues of the library polynucleotides to another base residue to obtain converted polynucleotides; optionally, wherein the converting comprises bisulfite conversion.

9. The method of any one of claims 1-8, wherein the conversion-resistant cytosine analog comprises a moiety that inhibits conversion to another base residue; optionally, wherein the conversion-resistant cytosine analog is selected from the group consisting of: 5-ethyl dCTP, 5-methyl dCTP, 5-fluoro dCTP, 5-bromo dCTP, 5-iodo dCTP, 5-chloro dCTP, 5-trifluoromethyl dCTP, 5-aza dCTP.

10. The method of any one of claims 1-9, further comprising sequencing the converted polynucleotides; optionally, further comprising:
(i) aligning sequences of the converted polynucleotides with a reference sequence;
(ii) aligning a sequence of a template strand with a sequence of a complementary strand; and/or
(iii) mapping a methylated cytosine residue on a sequence of a converted polynucleotide or a reference sequence.

## Patentansprüche

1. Verfahren zur Herstellung einer Polynukleotidbibliothek, umfassend:
(a) Erhalten einer Vielzahl doppelsträngiger Matrizennukleinsäuren und eines ersten Adapters, der eine Haarnadelstruktur und einen doppelsträngigen Bereich mit einem Nick oder einer nickbaren Stelle umfasst, wobei die nickbare Stelle ein Uracil oder ein Ribonukleotid umfasst;
(b) Anfügen des ersten Adapters an jedes Ende der doppelsträngigen Matrizennukleinsäuren mittels doppelsträngiger Ligation;
(c) Denaturieren der doppelsträngigen Matrizennukleinsäuren, um einzelsträngige Matrizennukleinsäuren mit einer Haarnadelstruktur zu erhalten;
(d) Verlängern der Haarnadelstruktur in Gegenwart eines konversionsresistenten Cytosinanalogons, um verlängerte Haarnadelstrukturen zu erhalten, die einen Matrizenstrang und einen komplementären Strang umfassen; und
(e) Anfügen eines Y-Adapters an ein Ende der verlängerten Haarnadelstrukturen mittels doppelsträngiger Ligation zum Erhalten von Bibliothekspolynukleotiden.

2. Verfahren nach Anspruch 1, wobei der doppelsträngige Bereich des ersten Adapters die nickbare Stelle umfasst.

3. Verfahren nach Anspruch 1 oder 2, wobei (c) das Inkontaktbringen der nickbaren Stelle mit einem Enzym vor der Denaturierung umfasst, wobei das Enzym ausgewählt ist aus einer Uracil-DNA-Glycosylase (UDG), einer DNA-Glycosylase-Lyase, einer RNase H oder einer Kombination davon.

4. Verfahren nach einem der Ansprüche 1 bis 3, wobei der Y-Adapter einen doppelsträngigen Abschnitt und einen nicht-komplementären Abschnitt mit einem ersten Einzelstrang und einem zweiten Einzelstrang umfasst; optional wobei der erste Einzelstrang und/oder der zweite Einzelstrang eine Sequenzierungsprimer-Bindungsstelle, eine Amplifikationsprimer-Bindungsstelle und/oder eine Zielstelle für eine Fangsonde umfasst.

5. Verfahren nach einem der Ansprüche 1-4, wobei der doppelsträngige Bereich des ersten Adapters eine Tag-Sequenz umfasst.

6. Verfahren nach Anspruch 5, wobei (a) das Erhalten einer Vielzahl der ersten Adapter umfasst, wobei sich die Tag-Sequenzen der Vielzahl der ersten Adapter voneinander unterscheiden.

7. Verfahren nach Anspruch 6, weiter umfassend das Identifizieren einer ersten Sequenz eines konvertierten Polynukleotids und einer zweiten Sequenz eines konvertierten Polynukleotids, die dieselbe Tag-Sequenz umfasst, durch Vergleichen der Tag-Sequenzen der konvertierten Polynukleotide, wodurch eine erste Sequenz eines konvertierten Polynukleotids und eine zweite Sequenz eines Bibliothekspolynukleotids identifiziert werden, die von derselben doppelsträngigen Matrizennukleinsäure abgeleitet sind.

8. Verfahren nach einem der Ansprüche 1 bis 7, weiter umfassend die Konversion von konversionssensitiven Cytosinresten der Bibliothekspolynukleotide in einen anderen Basenrest, um konvertierte Polynukleotide zu erhalten; optional wobei die Konversion eine Bisulfit-Umwandlung umfasst.

9. Verfahren nach einem der Ansprüche 1 bis 8, wobei das konversionsresistente Cytosin-Analogon eine Gruppe umfasst, die die Umwandlung in einen anderen Basenrest hemmt; optional, wobei das konversionsresistente Cytosin-Analogon ausgewählt ist aus der Gruppe bestehend aus: 5-Ethyl-dCTP, 5-Methyl-dCTP, 5-Fluor-dCTP, 5-Brom-dCTP, 5-lod-dCTP, 5-Chlor-dCTP, 5-Trifluormethyl-dCTP, 5-Aza-dCTP.

10. Verfahren nach einem der Ansprüche 1 bis 9, weiter umfassend die Sequenzierung der konvertierten Polynukleotide; optional weiter umfassend:
(i) Ausrichten der Sequenzen der konvertierten Polynukleotide an einer Referenzsequenz;
(ii) Ausrichten einer Sequenz eines Matrizenstrangs mit einer Sequenz eines komplementären Strangs; und/oder
(iii) Kartieren eines methylierten Cytosinrests auf einer Sequenz eines konvertierten Polynukleotids oder einer Referenzsequenz.

## Revendications

1. Procédé de préparation d'une banque de polynucléotides, comprenant :
(a) l'obtention d'une pluralité d'acides nucléiques matrices double brin, et d'un premier adaptateur comprenant une épingle à cheveux et une région double brin comprenant une coupure ou un site de coupure, dans lequel le site de coupure comprend un uracile ou un ribonucléotide ;
(b) la ligature du premier adaptateur à chaque extrémité des acides nucléiques matrices double brin par ligature double brin ;
(c) la dénaturation des acides nucléiques matrices double brin pour obtenir des acides nucléiques matrices simple brin comprenant une épingle à cheveux ;
d) l'extension de l'épingle à cheveux en présence d'un analogue de cytosine résistant à la conversion pour obtenir des épingles à cheveux étendues comprenant un brin matrice et un brin complémentaire ; et
(e) la ligature d'un adaptateur Y à une extrémité des épingles à cheveux étendues par ligature double brin pour obtenir des polynucléotides de banque.

2. Procédé selon la revendication 1, dans lequel la région double brin du premier adaptateur comprend le site de coupure.

3. Procédé selon la revendication 1 ou 2, dans lequel (c) comprend la mise en contact du site de coupure avec une enzyme avant la dénaturation, dans lequel l'enzyme est choisie parmi une glycosylase d'ADN d'uracile (UDG), une glycosylase-lyase d'ADN, une RNase H ou une combinaison de celles-ci.

4. Procédé selon l'une quelconque des revendications 1-3, dans lequel l'adaptateur en Y comprend une partie à double brin et une partie non complémentaire comprenant un premier brin simple et un second brin simple ; éventuellement, dans lequel le premier brin simple et/ou le second brin simple comprennent un site de liaison pour l'amorce de séquençage, un site de liaison pour l'amorce d'amplification, et/ou un site cible pour une sonde de capture.

5. Procédé selon l'une quelconque des revendications 1-4, dans lequel la région double brin du premier adaptateur comprend une séquence d'étiquettes.

6. Procédé selon la revendication 5, dans lequel (a) comprend l'obtention d'une pluralité du premier adaptateur, dans lequel les séquences d'étiquettes de la pluralité du premier adaptateur sont différentes les unes des autres.

7. Procédé selon la revendication 6, comprenant en outre l'identification d'une première séquence d'un polynucléotide converti et d'une seconde séquence d'un polynucléotide converti comprenant la même séquence d'étiquettes en comparant les séquences d'étiquettes des polynucléotides convertis, identifiant ainsi une première séquence d'un polynucléotide converti et une seconde séquence d'un polynucléotide de banque dérivé du même acide nucléique matrice double brin.

8. Procédé selon l'une quelconque des revendications 1-7, comprenant en outre la conversion de résidus de cytosine sensibles à la conversion des polynucléotides de banque en un autre résidu de base pour obtenir des polynucléotides convertis ; éventuellement, dans lequel la conversion comprend une conversion au bisulfite.

9. Procédé selon l'une quelconque des revendications 1-8, dans lequel l'analogue de cytosine résistant à la conversion comprend un fragment qui inhibe la conversion en un autre résidu de base ; éventuellement, dans lequel l'analogue de cytosine résistant à la conversion est choisi parmi le groupe constitué de : 5-éthyl dCTP, 5-méthyl dCTP, 5-fluoro dCTP, 5-bromo dCTP, 5-iodo dCTP, 5-chloro dCTP, 5-trifluorométhyl dCTP, 5-aza dCTP.

10. Procédé selon l'une quelconque des revendications 1-9, comprenant en outre le séquençage des polynucléotides convertis ; éventuellement, comprenant en outre :
(i) l'alignement de séquences des polynucléotides convertis avec une séquence de référence ;
(ii) l'alignement d'une séquence d'un brin matrice avec une séquence d'un brin complémentaire ; et/ou
(iii) le mappage d'un résidu de cytosine méthylé sur une séquence d'un polynucléotide converti ou une séquence de référence.
